# EUROPEAN PATENT SPECIFICATION

(11) **EP 1 793 809 B1**
(45) Date of publication and mention of the grant of the patent: **11.07.2012**
(21) Application number: 05805851.2
(22) Date of filing: 30.09.2005
(51) Int. Cl.: A61K 9/20, A61K 9/22, A61K 31/00, A61K 9/14

(54) **MODIFIED RELEASE IBUPROFEN DOSAGE FORM**
DOSIERUNGSFORM FÜR IBUPROFEN MIT MODIFIZIERTER FREISETZUNG
FORME POSOLOGIQUE D'IBUPROFENE A LIBERATION MODIFIEE

(30) Priority: 30.09.2004 US 614932 P; 10.06.2005 US 689631 P; 29.09.2005 US 238802
(43) Date of publication of application: 13.06.2007
(73) Proprietor: SCOLR Pharma, Inc., Bellevue, WA 98006 (US)
(72) Inventor: HITE, Michael, Seattle, WA 98118 (US); FEDERICI, Cathy, Seattle, WA 98122 (US); BRUNELLE, Alan, Woodinville, WA 98077 (US); TURNER, Stephen, Snoqualmie, WA 98065 (US)
(74) Representative: Schwabe - Sandmair - Marx
(86) International application number: PCT/US2005/035630
(87) International publication number: WO 2006/039692

(56) References cited:
- WO-A1-00/07570
- US-A- 5 009 895
- US-A- 5 100 675
- US-B1- 6 197 336
- US-B1- 6 361 794
- US-B1- 6 548 083

## Description

### BACKGROUND OF THE INVENTION

Ibuprofen is 2-(4-isobutylphenyl)propionic acid and is a non-steroidal anti-inflammatory compound (NSAID), which exhibits high levels of anti-inflammatory, analgesic and antipyretic activities necessary for the effective treatment of rheumatoid arthritis and osteo-arthritis and other inflammatory conditions. Most dosage forms of ibuprofen are immediate release dosage forms that provide rapid onset of therapeutic action, then rapidly declining levels of active ingredient, necessitating repeated dosing. They do not maintain therapeutic levels from one treatment over an extended period of time. Repeat dosing is thus required at intervals of four to six hours. WO 00/07570 discloses a stable pharmaceutical composition comprising a mixture of (i) an ibuprofen medicament; (ii) a domperidone medicament; and (iii) a carrier material characterized in that the carrier material is substantially free of povidone and comprises at least one diluent combined with at least one release modifying agent. The document states that when the dosage form is exposed to the aqueous medium after injection, the release modifying excipients cause the solid composition to release the active ingredient at the desired rate for example substantially immediately or at a desired controlled rate. Formulations that claim extended release fail to have an initial burst of the drug and thus exhibit substantial delay between administration and the achievement of an effective therapeutic blood level. Therefore, a need exists for a solid dosage form, for example a compressed tablet, which provides an initial burst of released ibuprofen, leading to prompt onset of action, then thereafter provides a sustained release of sufficient ibuprofen to maintain beneficial blood levels of ibuprofen over an extended period of 8 or more hours.

### SUMMARY OF THE INVENTION

In accordance with the foregoing, we have provided a solid dosage form for oral administration of ibuprofen comprising a modified release formulation of ibuprofen which provides an immediate burst effect and thereafter a sustained release of sufficient ibuprofen to maintain blood levels at least 6.4 µg/ml over an extended period of at least 8 hours following administration of a single dose.

More particularly, the invention relates to a solid dosage form for modified oral administration of ibuprofen comprising:
a hydrophilic polymer;
300 to 800 mg of ibuprofen in the solid dosage form uniformly dispersed in said polymer;
a dissolution additive dispersed in said hydrophilic polymer in an amount in the range of 10% to 35% by weight of the ibuprofen, said dissolution additive being sodium carbonate, glycine, arginine, croscarmellose sodium or a combination thereof, or a combination of any two of such dissolution additives; and
an inert formulation additive dispersed in said hydrophilic polymer in an amount in the range of 15% to 75% by weight of the ibuprofen, said formulation additive comprising microcrystalline cellulose, silica, magnesium stearate, stearic acid, lactose, pre-gelatinized starch, dicalcium phosphate or a combination of any of them,
wherein said hydrophilic polymer comprises a first hydroxypropyl methylcellulose having a viscosity of greater than 100 cps and a second HPMC having a viscosity of about 100 cps, each at a concentration of 17% to 42% by weight of ibuprofen,
wherein at least 20% of the ibuprofen is released within 2 hours following exposure to an agitated aqueous medium of a single dosage unit, then thereafter releases ibuprofen at a relatively constant rate over a period of at least 8 hours, and wherein at least 70% of the ibuprofen is released over a period of not more than 14 hours following such administration or exposure,
wherein the release is determined using an agitated aqueous medium, stirring at 50 rpm in pH 7.2 KH₂PO₄ media.

The dosage form releases ibuprofen at a rate sufficient to initially deliver a effective amount of ibuprofen within about 2.0 hours following administration. The dosage form then subsequently delivers the remaining amount of ibuprofen at a relatively constant rate sufficient to maintain a level of ibuprofen over a predetermined delivery period of for at least 8 hours.

As used herein, a relative constant rate refers to a substantially linear relationship shown in the examples following the initial burst (up to about 2 hours) between percentage released and elapsed time.

### BRIEF DESCRIPTION OF THE DRAWINGS

Figure 1: *In-vitro* dissolution of Example 1
Figure 2: *In-vitro* dissolution of Example 2
Figure 3: *In-vitro* dissolution of Example 3
Figure 4: *In-vitro* dissolution of Example 4
Figure 5: *In-vitro* dissolution of Example 5
Figure 6: *In-vitro* dissolution of Example 6
Figure 7: *In-vitro* dissolution of Example 7
Figure 8: *In-vitro* dissolution of Example 8
Figure 9: *In-vitro* dissolution of Example 9
Figure 10: *In-vitro* dissolution of Example 10
Figure 11: *In-vitro* dissolution of Example 11
Figure 12: *In-vitro* dissolution of Example 12
Figure 13: *In-vitro* dissolution of Example 13
Figure 14: *In-vitro* dissolution of Example 14
Figure 15: *In-vitro* dissolution of Example 15
Figure 16: *In-vitro* dissolution of Example 16
Figure 17: *In-vitro* dissolution of Examples 17 and 18
Figure 18: *In-vitro* dissolution of BRUFEN RETARD, an extended release form of Ibuprofen available for sale in Europe.
Figure 19: *In-vivo* data from comparison of present invention versus Motrin®

### DETAILED DESCRIPTION OF THE INVENTION

The present invention is further illustrated and described by reference to the following disclosure, examples and discussion below. In the examples and discussion which follow, the use of particular polymers, electrolytes, additives, fillers and tableting aids are provided by way of example only and are not intended to limit the scope of this invention. Although the invention is illustrated and described herein with reference to specific embodiments, the invention is not intended to be limited to the details shown. Rather, various modifications may be made in the details within the scope and range of equivalents of the claims and without departing from the invention.

The ibuprofen content of the dosage form is between in the range 300 mg and 800 mg per dosage unit, preferably about 300, 400 or 600 mg per unit dosage form. Also contemplated is using prodrugs of ibuprofen such as ibuprofen-lysine and ibuprofen-arginine. If a smaller dosage form is desired, a single dose of ibuprofen may be divided between multiple, for example two to three, dosage units, such as tablets, which may be administered at substantially the same time. The dosage form may comprise from about 25% to about 75% by weight ibuprofen.

These hydrophilic polymers used in the dosage form gel and dissolve slowly in aqueous acidic - media thereby allowing the ibuprofen to diffuse from the gel in the stomach and gastrointestinal tract. Hydroxypropyl methylcellulose (HPMC) are available in forms that have varying viscosity ratings. In general these polymers, or the combination of them, may be present in the dosage form alone or in combination in an amount or at a concentration in the range of 10% to 70% by weight of the ibuprofen present in the formulation, for example 15% to 50% or 15% to 33%, depending on the release pattern which is sought to be achieved with the particular dosage form.

One hydrophilic polymer useful in the present invention is HPMC K4M. This is a nonionic swellable hydrophilic polymer manufactured by "The Dow Chemical Company" under the tradename "Methocel." HPMC K4M is also referred to as HPMC K4MP, in which the "P" refers to premium cellulose ether designed for controlled release formulations. The "4" in the abbreviation suggests that the polymer has a nominal viscosity (2% in water) of 4000. The percent of methoxyl and hydroxypropyl groups are 19-24 and 7-12, respectively. In its physical form, HPMC K4M is a free-flowing, off-white powder with a particle size limitation of 90%<100 mesh screen. A more complete list of HPMC is K100LVP, K15MP, K100MP, E4MP and E10MP CR with nominal viscosities of 100, 15000, 100000, 4000, and 10000 respectively.

The solid dosage form also includes at least one formulation additive such as one or more of a filler, a diluent or a compression aid. These are additives which aid in preparation or manufacture of the dosage form and for a tableted solid dosage form a tableting aid such as microcrystalline cellulose (MCC), such MCC 105 (particle size of about 20 µm), MCC 200 (particle size of about 180 µm) and MCC 302 (particle size of about 90 µm), silicified microcrystalline cellulose (MCC bonded to SiO₂), such as Prosolv90 (particle size of about 90 µm) and Prosolv50 (paricle size of about 50 µm), lactose, such as spray dried lactose (Lactopress^{®}), dicalcium phosphate, silica or pregelatinized starch and combinations thereof are incorporated into the formulation in an amount or at a concentration in the range of about 15% to about 75% by weight of the ibuprofen present in the dosage form. It is contemplated that various particle sizes of microcrystalline cellulose may be used if desired, for example two different particle sizes in which each of them are present in individual amounts in the range of 17% to 33% by weight of the ibuprofen present in the formulation. In one embodiment, one can pre-blend silica with ibuprofen or pre-blend silica and/or formulation additive MCC with ibuprofen.

In addition to formulation additives, the dosage form also contains at least one dissolution additive. Such additives which generally comprise a pore-forming, wetting or disintegration agent which facilitates dissolution of the dosage form. Such dissolution additives are present in the dosage form at an amount or concentration in the range of about 10% to about 35% by weight of the ibuprofen, for example, at 10% to about 15%. The additive is suitably selected from sodium carbonate, glycine, arginine and croscarmellose sodium or a combination thereof, or a combination of any two of such dissolution additives.

In addition to ibuprofen, multiple active ingredients are contemplated and may be present in the present dosage form. Combinations of ibuprofen with actives such as caffeine, psuedophedrine, aspirin, phenylephrine and/or sympathomemetics, analgesics, such as hydrocodone, and antihistamines are within the scope of the invention.

Favorable *in vitro* characteristics that lead to an acceptable *in vivo* efficacy are contemplated as 20% or greater release within 2.0 hour after oral administration or contact with an aqueous environment, followed by more gradual release over several hours, leading to release of at least 70% release in 8 to 12 hours following administration or contact with an aqueous environment. The method of determining in *vitro* release is using an agitated aqueous medium, such as stirring at 50 rpm in pH 7.2 KH₂PO₄ media; or surrogate methods using alternate pH media, such as 0.1N HCl or SGF @ pH 1.2 for an initial (30min-2hr period or using alternate hydrodynamic conditions such as 100 to 150rpm for a period of 1-2hrs).

The accepted range for minimal efficacy *in vivo* is from about 6.4 µg/ml to about 10 µg/ml mean ibuprofen blood concentration.

### Examples

The formulations of the invention are illustrated by the following examples. The use of particular polymers, electrolytes, additives, fillers and compression aids are not intended to limit the scope of this invention but are exemplary only.

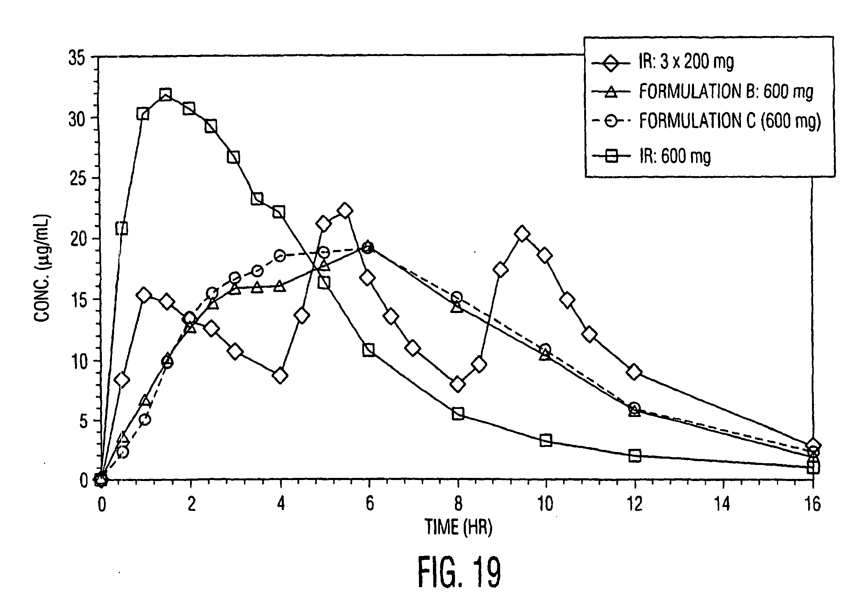

The solid dosage comprising a modified release formulation of the present invention was prepared and tested for both *in vitro* release and *in vivo* blood levels as described in Examples 1-20 below. In the *in vivo* testing, the dissolution rates of the subject dosage forms were compared against two commercially available tablets, one being an immediate release formulation of 200 mg of ibuprofen and the other being an immediate release 600 mg ibuprofen formulation. The solid dosage forms comprising the modified release formulation of the present invention demonstrated an initial burst similar to an immediate release tablet and a slower, more controlled release of ibuprofen over a eight hour period, as best seen in Fig. 19.

Unless otherwise noted, all *in vitro* release performance was evaluated in a type II dissolution apparatus in 900mL KH₂PO₄ buffer, pH 7.2, at 50rpm paddle speed.

### Example 1

In one embodiment, the formulation comprised ibuprofen, hydroxypropyl methylcellulose (HPMC K15M and HPMC K100LV), glycine and sodium carbonate, in which HPMC K15M was present at a concentration of 18% by weight of ibuprofen, HPMC K100LV was present at a concentration of 17% by weight of ibuprofen, glycine was present at a concentration of 2.5% by weight of ibuprofen, and sodium carbonate was present at a concentration of 17% by weight of ibuprofen within a monolithic compressed tablet. The specific formulations are as follows:

| **Ex.1a** | **mg** | | **Ex.1b** | **mg** |
|---|---|---|---|---|
| Ibuprofen 90 grade | 600 | | Ibuprofen 90 grade | 600 |
| HPMC K15M | 110 | | HPMC K15M | 125 |
| HPMC K100LV | 100 | | HPMC K100LV | 100 |
| MCC PH102 | 100 | | MCC PH102 | 100 |
| Na₂CO₃, anhydrous | 150 | | Na₂CO₃, anhydrous | 150 |
| Glycine | 15 | | Glycine | 15 |
| Silica, Syloid 244 | 20 | | Silica, Syloid 244 | 20 |
| Mg Stearate | 10 | | Mg Stearate | 10 |
| Total: A | 1105 | | Total: | 1120 |

Ingredients were passed through a 30-mesh screen and blended with the remaining formulation components in a V-blender. The resulting powder was compressed into tablets using conventional compression techniques.

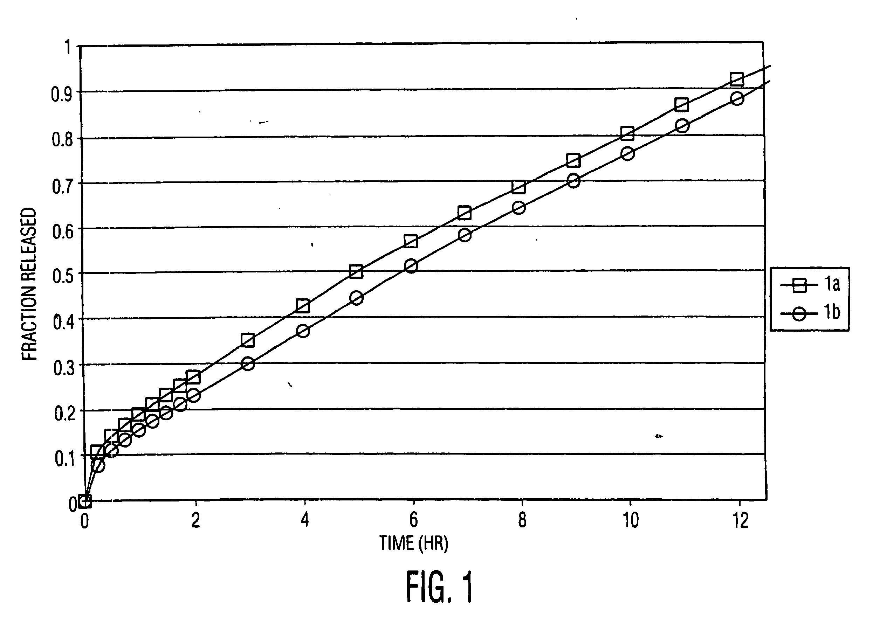

As shown in **Fig. 1****,** the results of this Example demonstrate that the invention is capable of an *in vitro* release profile comprising a burst effect, followed by the sustained release of the remaining material, leading to in excess of 90% release in approximately 12 hours. This formulation thus overcomes one of the principle problems with many ibuprofen formulations which exhibit substantially less than complete release over an extended period of time.

### Example 2

In another embodiment, the formulation comprised ibuprofen, hydroxypropyl methylcellulose (HPMC K100M and HPMC K100LV), sodium carbonate, flow agents and tableting aids, in which HPMC K100M was present at a concentration of 17% by weight of ibuprofen, HPMC K100LV was present at a concentration of 17% by weight of ibuprofen and sodium carbonate was present at a concentration of 25% by weight of ibuprofen within a compressed monolithic tablet. The specific formula is as follows:

| **Ex. 2** | **mg** |
|---|---|
| Ibuprofen | 600 |
| HPMC K100M | 100 |
| HPMC K100LV | 100 |
| Na₂CO₃, anhydrous | 150 |
| MCC PH102 | 150 |
| Silica, Syloid 244 | 20 |
| Mg Stearate | 10 |
| Total: | 1130 |

The formulation components were mixed in a V-blender. The resulting powder was compressed into tablets using conventional technologies. In this Example a combination of a medium to high viscosity HPMC and a low viscosity HPMC was used.

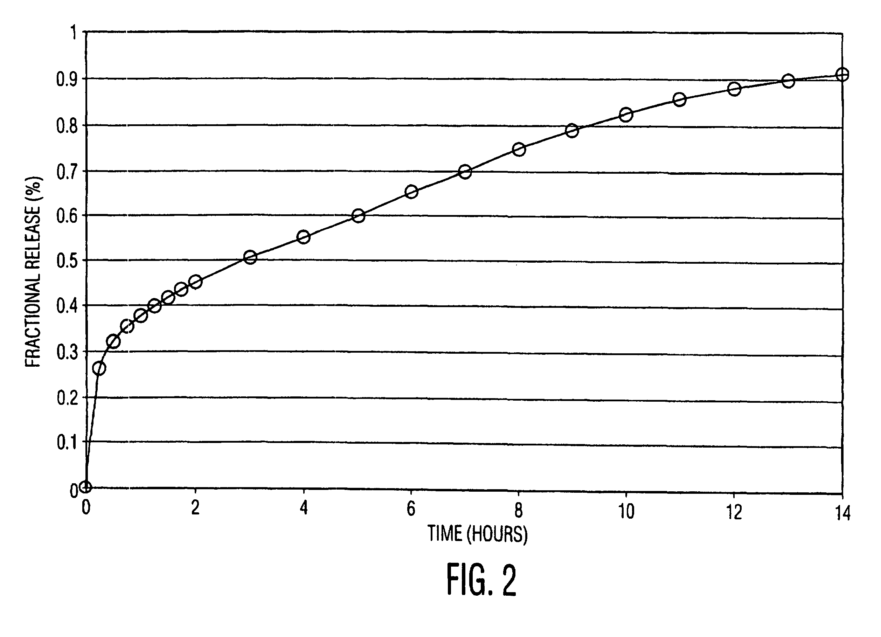

As shown in **Fig. 2**, the results of this Example demonstrate an *in vitro* release profile comprising a burst effect, followed by the sustained release of the remaining material. The burst effect provides release of 20% of ibuprofen within 2 hours, and the release of approximately 90% of the available ibuprofen over a period of 12 to 14 hours.

### Example 3

In another embodiment, the formulation comprised ibuprofen, hydroxypropyl methylcellulose (HPMC K15M and HPMC K100LV), sodium carbonate, flow agents and tableting aids, in which HPMC K100M was present at a concentration of 17% by weight of ibuprofen, HPMC K100LV was present at a concentration of 17% by weight of ibuprofen and sodium carbonate was present at a concentration of 25% by weight of ibuprofen within a compressed monolithic tablet.

| **Ex. 3** | **mg** |
|---|---|
| Ibuprofen | 600 |
| HPMC K15M | 100 |
| HPMC K100LV | 100 |
| MCC PH102 | 100 |
| Na₂CO₃, anhydrous | 150 |
| Glycine | 15 |
| Silica, Syloid 244 | 20 |
| Mg Stearate | 10 |
| Total: | 1095 |

The formulation components were mixed in a V-blender. The resulting powder was compressed into tablets using conventional compression technology. In this Example a combination of a medium to high viscosity HPMC and a low viscosity HPMC was used.

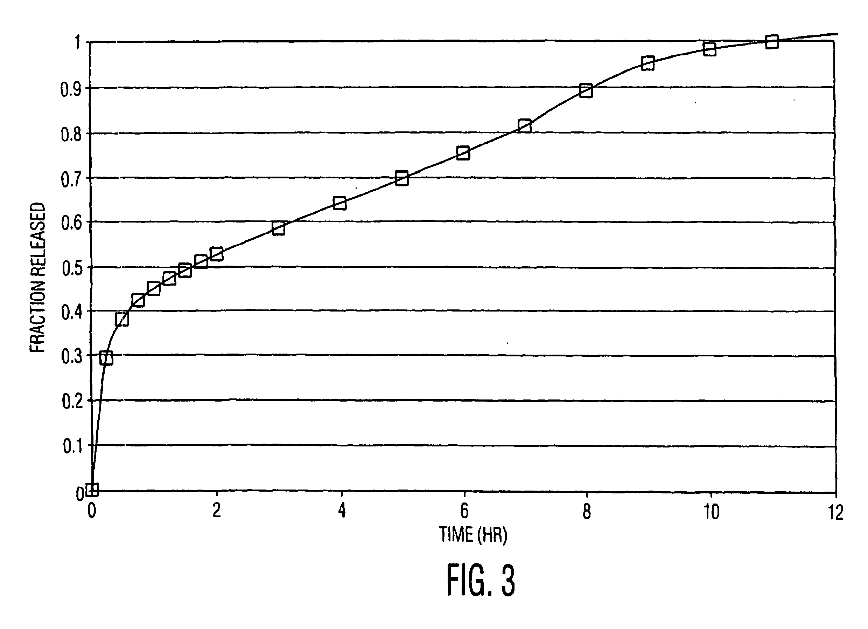

As shown in **Fig. 3**, the results of this Example demonstrate an *in vitro* release profile comprising a burst effect providing release of 20% of ibuprofen within 2 hours, followed by the sustained release of the remaining material evidencing release of 100% of the ibuprofen present in about 11 hours and greater than 90% in approximately 8 hours.

### Example 4

In another embodiment, the formulation comprised ibuprofen, hydroxypropyl methylcellulose (HPMC K100M and HPMC K100LV), sodium carbonate, flow agents and tableting aids, in which HPMC K100M was present at a concentration of 17% by weight of ibuprofen, HPMC K100LV was present at a concentration of 17% by weight of ibuprofen, and sodium carbonate was present at a concentration of 25% by weight of ibuprofen within a compressed monolithic tablet.

| **Ex. 4** | **mg** |
|---|---|
| Ibuprofen | 600 |
| HPMC K100M | 100 |
| HPMC K100LV | 100 |
| MCC PH102 | 100 |
| Na₂CO₃, anhydrous | 150 |
| Silica, Syloid 244 | 20 |
| Mg Stearate | 10 |
| Total: | 1080 |

The formulation components were mixed in a V-blender. The resulting powder was compressed into tablets using conventional technologies. In this Example a combination of a medium to high viscosity HPMC and a low viscosity HPMC was used.

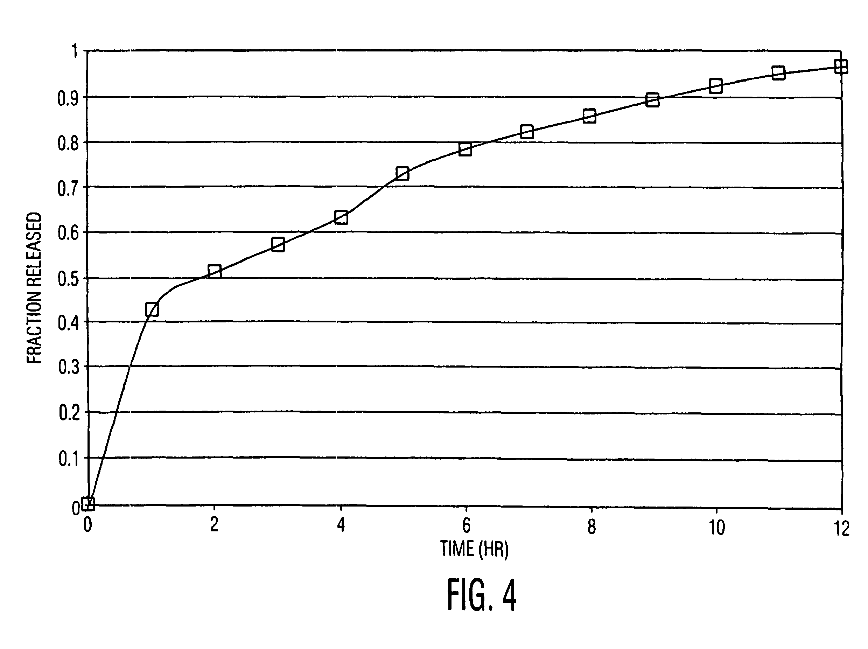

As shown in **Fig. 4**, the results of this Example demonstrate an *in vitro* release profile comprising a burst effect, followed by the sustained release of the remaining material. 20% of ibuprofen was released within 2 hours, followed by gradual sustained release, resulting in approximately 95% release after 12 hours.

### Example 5 (not according to the invention)

In another embodiment, the formulation comprised ibuprofen, hydroxypropyl methylcellulose (HPMC K100M), polyethylene oxide (PEO WSRN 301), sodium carbonate, glycine, flow agents and tableting aids, in which HPMC was present at a concentration of 33% by weight of ibuprofen, glycine was present at a concentration of 8.25% by weight of ibuprofen and sodium carbonate was present at a concentration of 25% by weight of ibuprofen within a compressed monolithic tablet.

| **Ex. 5** | **mg** |
|---|---|
| Ibuprofen | 600 |
| PEO 301 | 50 |
| HPMC K100M | 100 |
| MCC PH102 | 100 |
| Na₂CO₃, anhydrous | 150 |
| Glycine | 20 |
| Silica, Syloid 244 | 20 |
| Mg Stearate | 10 |
| Total: | 1050 |

The formulation components were mixed in a V-blender. The resulting powder was compressed into tablets using conventional compression technology.

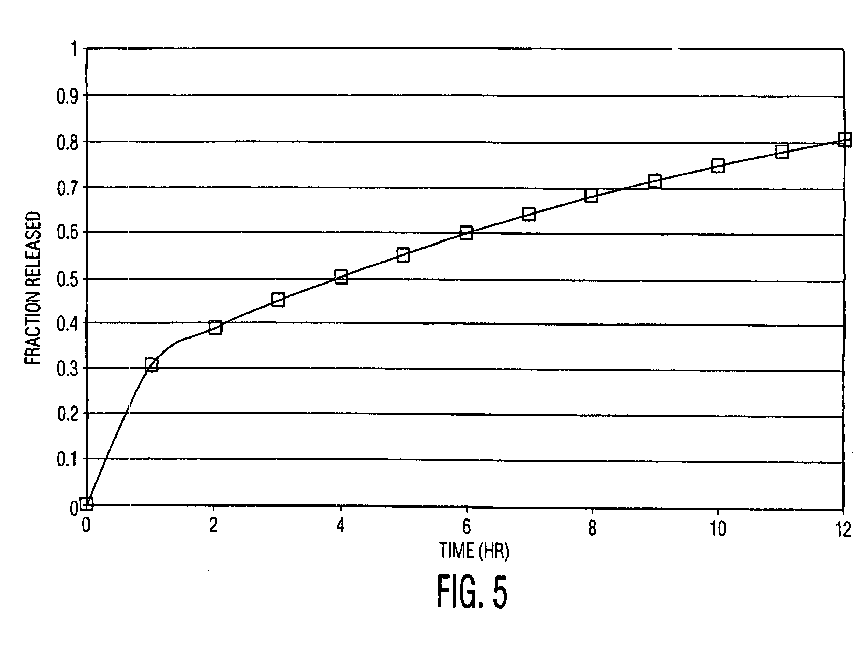

As shown in **Fig. 5**, the results of this Example demonstrate an *in vitro* release profile comprising a burst effect, followed by the sustained release of the remaining material. For this formulation 20% of ibuprofen was released within 2 hours, but incomplete release was evidenced after 12 hours.

### Example 6 (not according to the invention)

In another embodiment, the formulation comprised ibuprofen, hydroxypropyl methylcellulose (HPMC K15M), potassium carbonate, flow agents and tableting aids, in which HPMC was present at a concentration of 33% by weight of ibuprofen, and potassium carbonate was present at a concentration of 17% by weight of ibuprofen within a compressed monolithic tablet.

| **Ex. 6** | **mg** |
|---|---|
| Ibuprofen 90 grade | 600 |
| MCC PH 105 | 210 |
| HPMC K15M Prem | 190 |
| MCC PH 200 | 100 |
| K₂CO₃ anhydrous | 100 |
| | 1200 |

The formulation components were mixed in a V-blender. The resulting powder was compressed into tablets using conventional compression technology.

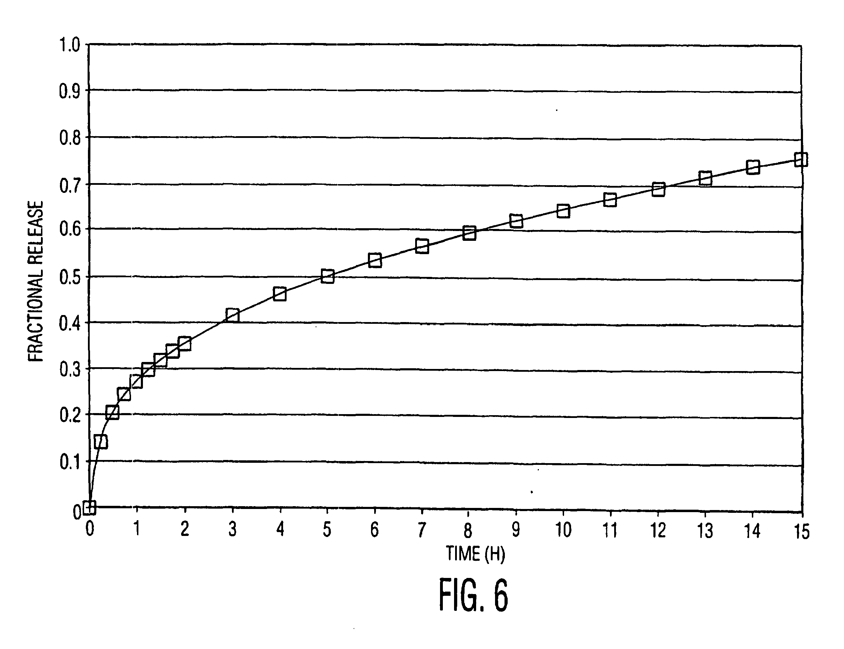

As shown in **Fig. 6**, the results of this Example demonstrate an *in vitro* release profile comprising a burst effect, followed by the sustained release of the remaining material. 20% of ibuprofen was released in under 2 hours, and release was thereafter sustained over a period of 15 hours. However, incomplete release was exhibited by the dosage form.

### Example 7 (not according to the invention)

In this embodiment, the formulation comprised ibuprofen, hydroxypropyl methylcellulose (HPMC K15M), sodium carbonate, microcrystalline cellulose (MCC PH105 and MCC PH200), in which HPMC was present at a concentration of 33% by weight of ibuprofen, sodium carbonate was present at a concentration of 17% by weight of ibuprofen, MCC PH105 was present at a concentration of 33%, and MCC PH200 was present at a concentration of 17% within a compressed monolithic tablet.

| **Ex. 7** | **Mg** |
|---|---|
| Ibuprofen 90 grade | 600 |
| HPMC K15M Prem | 190 |
| MCC PH 105 | 210 |
| MCC PH 200 | 100 |
| Na₂CO₃ anhydrous | 100 |
| | 1200 |

All ingredients were passed through a 30-mesh screen. The ibuprofen and the MCC 105 were blended in a V-blender. The resulting homogenous pre-blend was granulated with water, dried and subsequently blended with the remaining formulation components in a V-blender. The resulting powder was compressed into tablets using conventional compression technology.

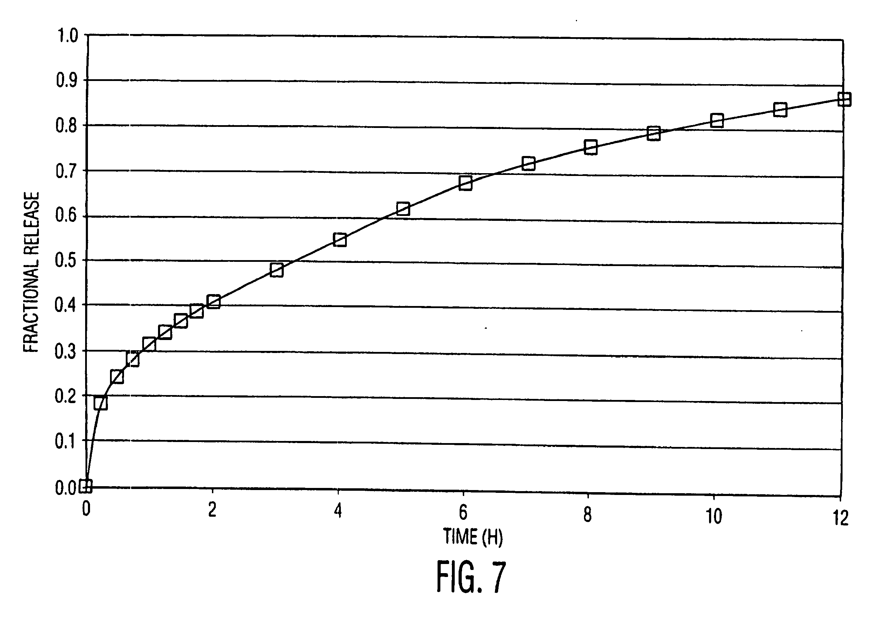

As shown in **Fig. 7**, this Example demonstrates an *in vitro* release profile comprising a burst effect, followed by the sustained release of the remaining material. The burst effect releases 20% of ibuprofen in under 2 hour, followed by relatively constant release over the next 10 -12 hours and resulting in approximately 90% release after 12 hours.

### Example 8

In the embodiment of Example 1, the tablet resulting from the formulation was split into two equal parts, and both sections were placed into a dissolution vessel.

| **Ex. 8** | **mg** |
|---|---|
| Ibuprofen 90 grade | 600 |
| HPMC K15M | 110 |
| HPMC K100LV | 100 |
| MCC PH102 | 100 |
| Na₂CO₃, anhydrous | 150 |
| Glycine | 15 |
| Silica, Syloid 244 | 20 |
| Mn Stearate | 10 |
| Total: | 1105 |

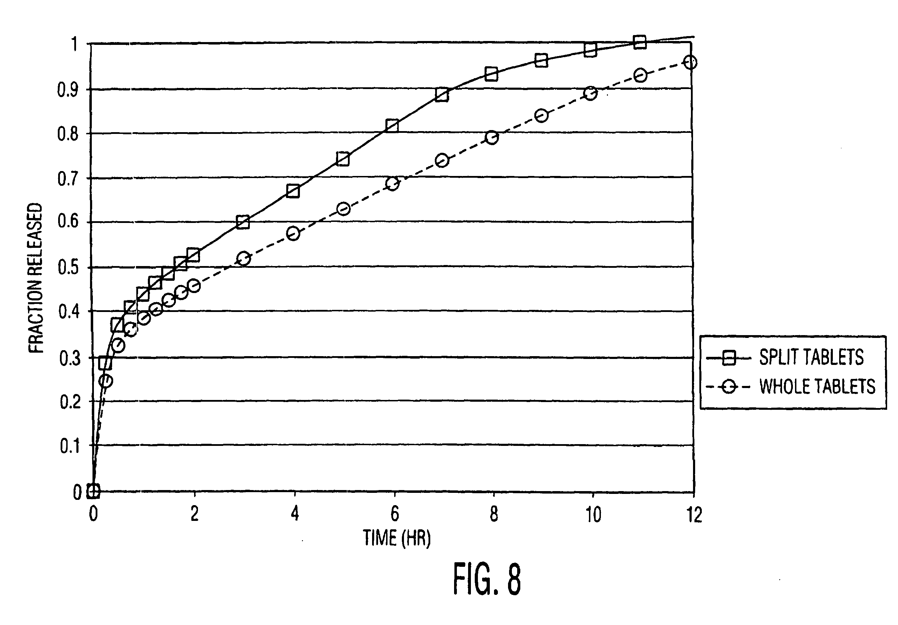

As shown in **Fig. 8**, the results of this Example demonstrates an *in vitro* release profile comprising a burst effect, followed by the sustained release of the remaining material, even when split into sections after tableting. In each case 20% of ibuprofen was released in less than one hour and substantially all the ibuprofen had been released at about 12 hours.

### Example 9 (not according to the invention)

In one embodiment, the formulation comprised ibuprofen, hydroxypropyl methylcellulose (HPMC K15M), sodium carbonate, microcrystalline cellulose (MCC PH 302), in which HPMC was present at a concentration of 33% by weight of ibuprofen, sodium carbonate was present at a concentration of 18% by weight of ibuprofen, and MCC PH 302 was present at a concentration of 33% within a compressed monolithic tablet.

| **Ex. 9** | **mg** |
|---|---|
| Ibuprofen 90 grade | 300 |
| HPMC K15M Prem | 100 |
| MCC PH 302 | 100 |
| Na₂CO₃ anhydrous | 50 |
| Glycine | 7.5 |
| Silica | 5.5 |
| Total: | 563 |

All ingredients were passed through a 30-mesh screen and blended in a V-blender. The resulting homogenous pre-blend was granulated with water, dried and subsequently blended with the remaining formulation components in a V-blender. The resulting powder was compressed into tablets using conventional technologies.

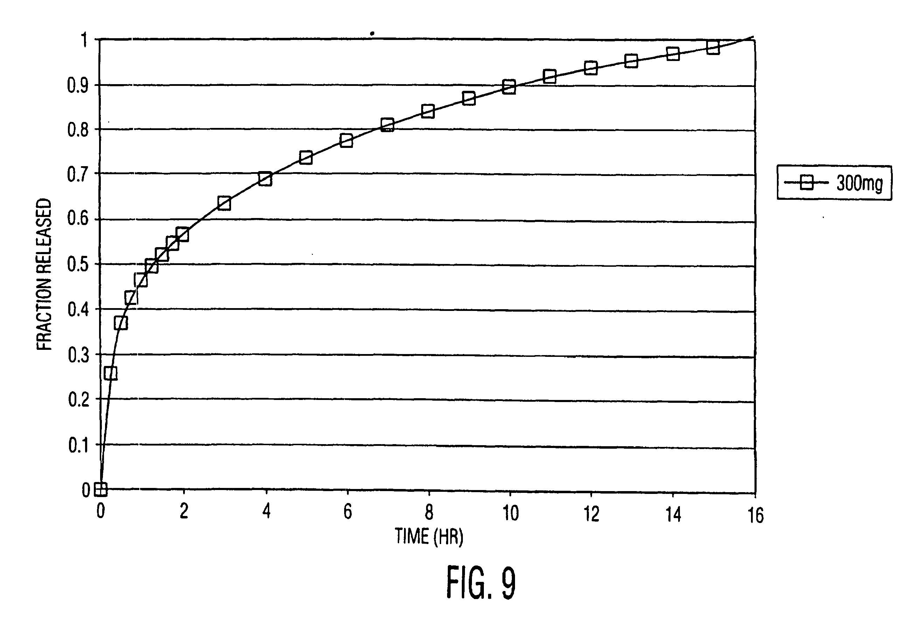

As shown in **Fig. 9**, the results of this Example demonstrate an *in vitro* release profile comprising a burst effect, followed by the sustained release of the remaining material. 20% of ibuprofen was released within 2 hours, about 90% release was obtained in about 9 hours followed by 100% release in under 16 hours.

### Example 10 (not according to the invention)

In another embodiment, the formulation comprised ibuprofen, hydroxypropyl methylcellulose (HPMC K4M), flow agents and tableting aids, in which HPMC K4M was present at a concentration of 32% by weight of ibuprofen, and arginine was present at a concentration of 17% by weight of ibuprofen within a compressed monolithic tablet.

| **Ex. 10** | **mg** |
|---|---|
| Ibuprofen 90 grade | 600 |
| Silica | 5.5 |
| MCC PH 105 | 210 |
| HPMC K4M Prem | 190 |
| Arginine | 100 |
| Silica | 5.5 |
| Total: | 1111 |

The formulation components were mixed in a V-blender. The resulting powder was compressed into tablets using conventional technologies.

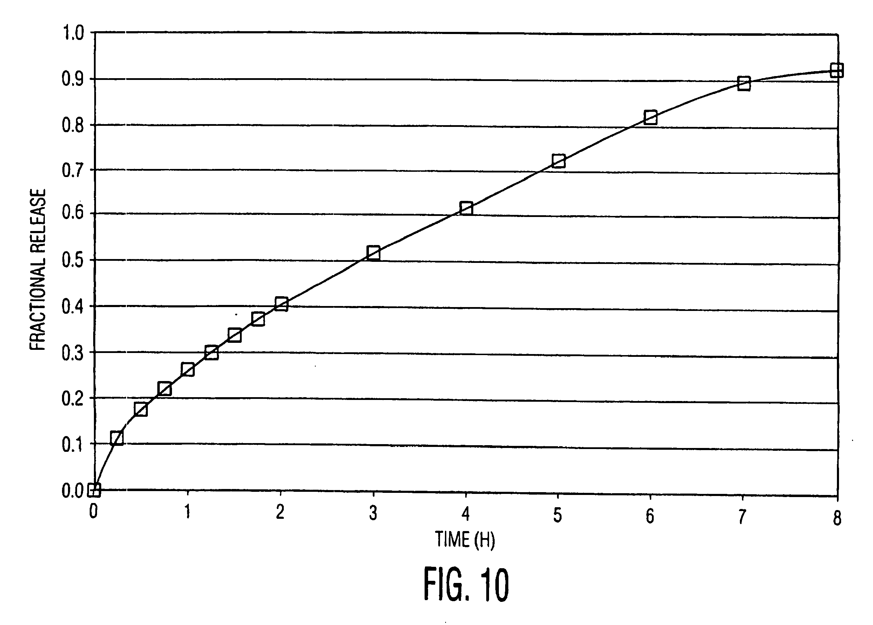

As shown in **Fig. 10**, the results of this Example demonstrate an *in vitro* release profile comprising a slight burst effect, followed by the sustained release of the remaining material. While the burst effect in this formulation produces somewhat delayed achievement of the percentage released, this formulation demonstrates in excess of 90% release over a period of 8 hours. (not according to the invention)

### Example 11 (not according to the invention)

In another embodiment, the formulation comprised ibuprofen, hydroxypropyl methylcellulose (HPMC K4M), sodium carbonate, arginine, flow agents and tableting aids, in which HPMC K4M was present at a concentration of 32% by weight of ibuprofen, sodium carbonate was present at concentration of 17% by weight of the ibuprofen, and arginine was present at a concentration of 17% by weight of ibuprofen within a compressed monolithic tablet.

| **Ex. 11** | **mg** |
|---|---|
| Ibuprofen 90 grade | 600 |
| Silica | 5.5 |
| MCC PH 105 | 210 |
| HPMC K4M Prem | 190 |
| Na₂CO₃ anhydrous | 100 |
| MCC PH 200 | 100 |
| Arginine | 100 |
| Silica | 5.5 |
| Stearic Acid | 12 |
| Total: | 1323 |

The formulation components are mixed in a V-blender. The resulting powder was compressed into tablets using conventional technologies.

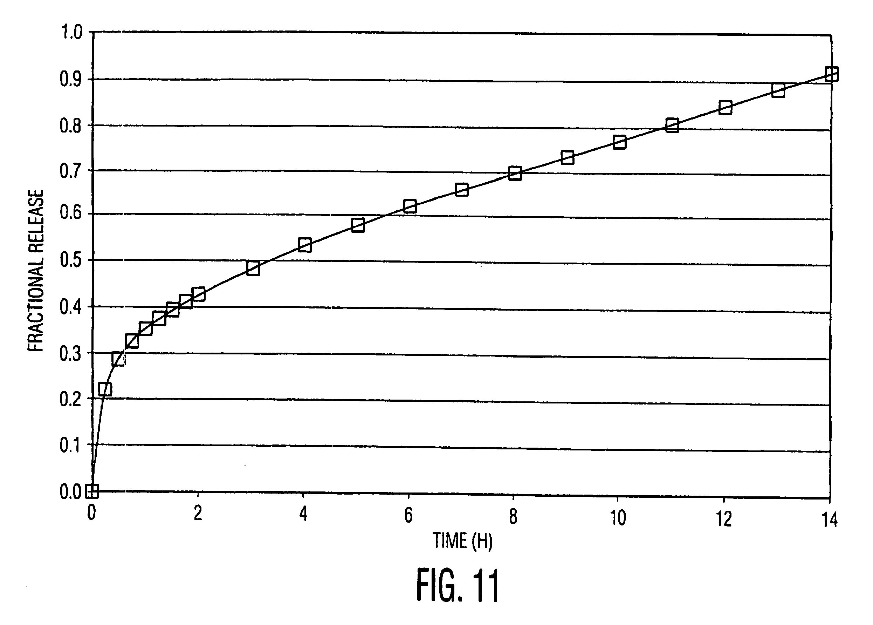

As shown in **Fig. 11**, the results of this Example demonstrate the *in vitro* release profile comprising a burst effect, followed by the sustained release of the remaining material. The initial release is greater than 20% of ibuprofen in less than two hours, and approximately 90% release over a period of 14 hours.

### Example 12 (not according to the invention)

In another embodiment, the formulation comprised ibuprofen, hydroxypropyl methylcellulose (HPMC K4M), microcrystalline cellulose (MCC 105), sodium carbonate, flow agents and various tableting aids, in which HPMC K4M was present at a concentration of 32% by weight of ibuprofen, sodium carbonate was present at concentration of 17% by weight of the ibuprofen, and tableting aid, either Lactopress (12a), dicalcium phosphate (12b), or pregelatinized starch (12c), was present at a concentration of 17% by weight of ibuprofen within a monolithic tablet.

| **Ex. 12a** | **mg** | | **Ex. 12b** | **mg** |
|---|---|---|---|---|
| Ibuprofen 90 grade | 600 | | Ibuprofen 90 grade | 600 |
| Silica | 5.5 | | Silica | 5.5 |
| MCC PH 105 | 210 | | MCC PH 105 | 210 |
| HPMC K4M Prem | 190 | | HPMC K4M Prem | 190 |
| Na₂CO₃ anhydrous | 100 | | Na₂CO₃ anhydrous | 100 |
| Lactopress | 100 | | Dicalcium phosphate | 100 |
| Silica | 5.5 | | Silica | 5.5 |
| Stearic acid | 12 | | Stearic acid | 12 |
| Total: | 1223 | | Total: | 1223 |

| **Ex. 12c** | **mg** | | | |
|---|---|---|---|---|
| Ibuprofen 90 grade | 600 | | | |
| Silica | 5.5 | | | |
| MCC PH 105 | 210 | | | |
| HPMC K4M Prem | 190 | | | |
| Na₂CO₃ anhydrous | 100 | | | |
| Starch 1500 | 100 | | | |
| Silica | 5.5 | | | |
| Stearic acid | 12 | | | |
| Total: | 1223 | | | |

All ingredients were passed through a 30-mesh screen. The ibuprofen and the MCC 105 were blended in a V-blender. The resulting homogenous pre-blend was granulated with water, dried and subsequently blended with the remaining formulation components in a V-blender. The resulting powder was compressed into tablets using conventional technologies.

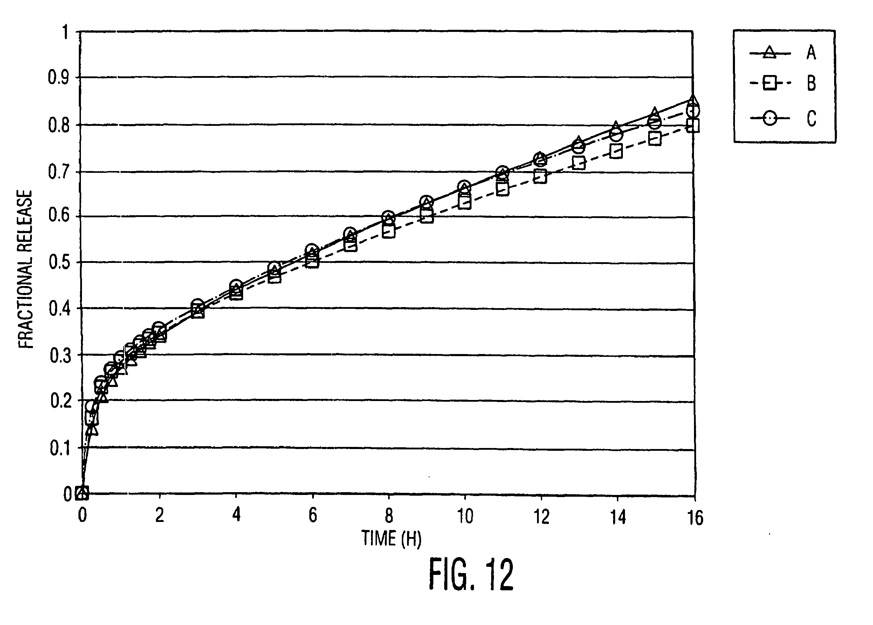

As shown in **Fig. 12**, the results of this Example demonstrate the invention is capable of an *in vitro* release profile comprising a burst effect, followed by the sustained release of the remaining material, with little or no alteration in release profile when the tableting aid selection is varied. The *in vitro* profile shows greater than 20 % release before 2.0 hours with a constant rate release and at least 70% release by 14 hours.

### Example 13 (not according to the invention)

In another embodiment, the formulation comprised ibuprofen, hydroxypropyl methylcellulose (HPMC K4M), microcrystalline cellulose (MCC 105), sodium carbonate, flow agents and various tableting aids, in which HPMC K4M was present at a concentration of 32% by weight of ibuprofen, sodium carbonate was present at concentration of 17% by weight of the ibuprofen, and croscarmellose sodium was present at a concentration of 3% by weight of ibuprofen within a monolithic tablet.

| **Ex. 13** | **mg** |
|---|---|
| Ibuprofen 90 grade | 600 |
| Silica | 5.5 |
| MCC PH 105 | 210 |
| HPMC K4M Prem | 190 |
| Na₂CO₃ anhydrous | 100 |
| MCC PH 200 | 100 |
| Croscarmellose sodium | 18 |
| Silica | 5.5 |
| Stearic acid ∼ 1% | 12 |
| Total: | **1241** |

All ingredients were passed through a 30-mesh screen. The ibuprofen, silica and the MCC 105 were blended in a V-blender. The resulting homogenous pre-blend was granulated with water, dried and subsequently blended with the remaining formulation components in a V-blender. The resulting powder was compressed into tablets using conventional technologies.

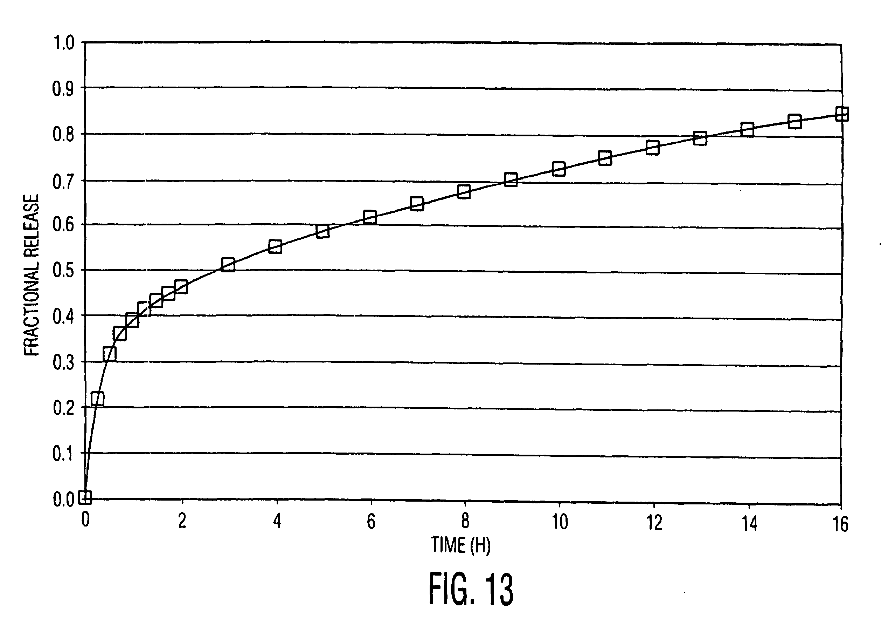

As shown in **Fig. 13**, the results of this Example demonstrates an *in vitro* release profile comprising a burst effect, followed by the sustained release of the remaining material. The *in vitro* profile shows greater than 20% release before 2.0 hours followed by a relatively constant rate release and at least 80% release by 14 hours.

### Example 14 (not according to the invention)

In another embodiment, the formulation comprised ibuprofen, hydroxypropyl methylcellulose (HPMC K4M), microcrystalline cellulose (MCC 105), glycine, sodium carbonate, flow agents and various tableting aids, in which HPMC K4M was present at a concentration of 32% by weight of ibuprofen, sodium carbonate was present at concentration of 17% by weight of the ibuprofen, glycine was present at a concentration of 8% by weight of ibuprofen and croscarmellose sodium was present at a concentration of 6% by weight of ibuprofen within a monolithic tablet.

| **Ex. 14** | **mg** |
|---|---|
| Ibuprofen 90 grade | 600 |
| MCC PH 105 | 200 |
| Silica | 5.5 |
| HPMC K4M Prem | 190 |
| MCC PH 200 | 100 |
| Glycine | 50 |
| Croscarmellose sodium | 35 |
| Silica | 5.5 |
| Stearic acid ∼ 1% | 12 |
| Total: | 1198 |

All ingredients were passed through a 30-mesh screen. The ibuprofen, silica and the MCC 105 were blended in a V-blender. The resulting homogenous pre-blend was granulated with water, dried and subsequently blended with the remaining formulation components in a V-blender. The resulting powder was compressed into tablets using conventional technologies.

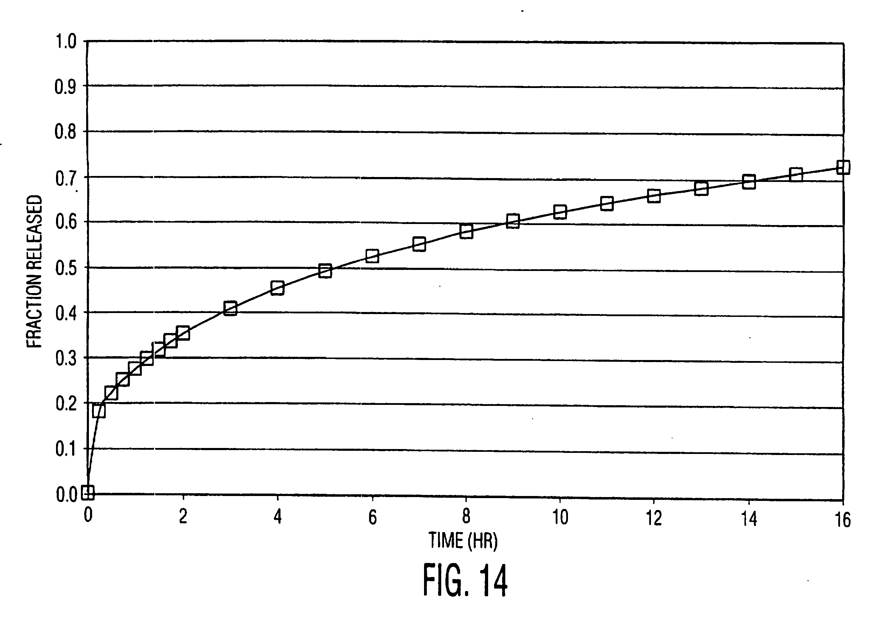

As shown in **Fig. 14**, the results of this Example demonstrate the invention is capable of an *in vitro* release profile comprising a burst effect, followed by the sustained release of the remaining material. The *in vitro* profile shows greater than 20% release before 2.0 hours with a constant rate release and at least 70% release by 14 hours.

### Example 15

In another embodiment, the formulation comprised ibuprofen, polyethylene oxide (PEO 301), PEO 60K, glycine, sodium carbonate, flow agents and various tableting aids, in which PEO was present at a concentration of 32% by weight of ibuprofen, sodium carbonate was present at concentration of 25% by weight of the ibuprofen, and glycine was present at a concentration of 37% by weight of ibuprofen within a monolithic tablet.

| **Ex. 15** | **mg** |
|---|---|
| Ibuprofen | 400 |
| PEO 301 | 50 |
| PEO 60K | 75 |
| Na2CO3 | 100 |
| Glycine | 150 |
| Maltodextrin M-580 | 100 |
| Stearic acid | 10 |
| Silica | 10 |
| **Total:** | **895** |

All ingredients were passed through a 30-mesh screen. The ibuprofen was blended with the formulation components in a V-blender. The resulting powder was compressed into tablets using conventional technologies.

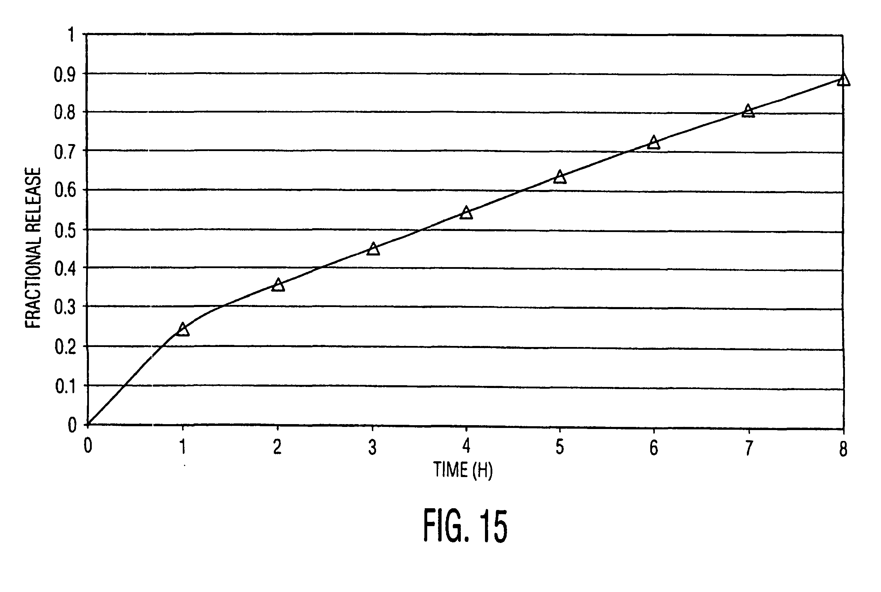

As shown in **Fig. 15**, the results of this Example demonstrate the invention is capable of an *in vitro* release profile comprising a burst effect, followed by the sustained release of the remaining material. The *in vitro* profile shows greater than 20% release before 2.0 hours with a constant rate release and at least 80% release by 8 hours.

### Example 16 (not according to the invention)

In another embodiment, the formulation comprised ibuprofen, polyethylene oxide (PEO 301), PEO 60K, glycine, sodium carbonate, flow agents and various tableting aids, in which PEO was present at a concentration of 32% by weight of ibuprofen, sodium carbonate was present at concentration of 25% by weight of the ibuprofen, and glycine was present at a concentration of 37% by weight of ibuprofen within a monolithic tablet.

| **Ex. 16** | **mg** |
|---|---|
| Ibuprofen | 400 |
| PEO 301 | 50 |
| PEO 60K | 50 |
| Na2CO3 | 100 |
| Glycine | 100 |
| Maltodextrin M-580 | 100 |
| Stearic acid | 10 |
| Silica | 10 |
| **Total:** | **820** |

All ingredients were passed through a 30-mesh screen. The ibuprofen was blended with the formulation components in a V-blender. The resulting powder was compressed into tablets using conventional technologies.

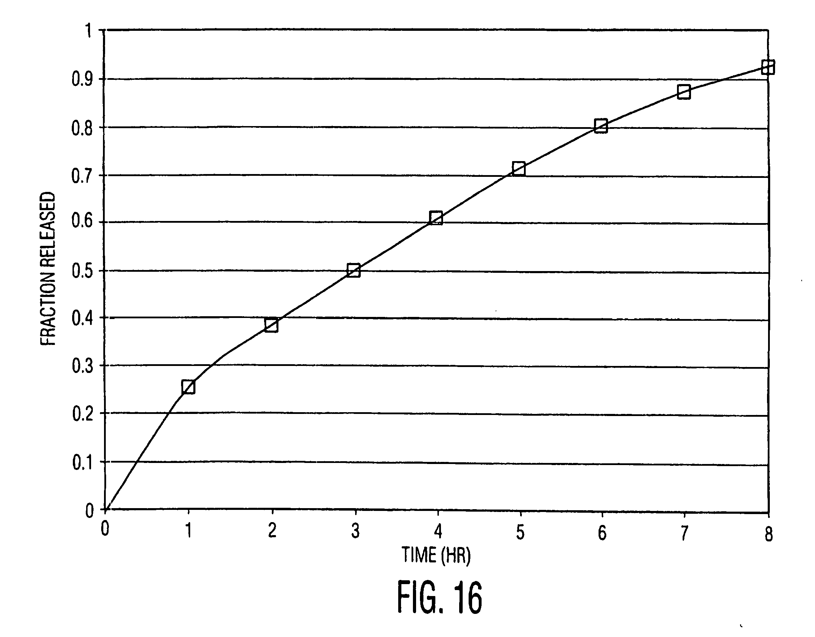

As shown in **Fig. 16**, the results of this Example demonstrate the invention is capable of an *in vitro* release profile comprising a burst effect, followed by the sustained release of the remaining material. The *in vitro* profile shows greater than 20 % release before 2.0 hours with a constant rate release and at least 90% release by 8 hours.

### Example 17 (not according to the invention)

In another embodiment, the formulation comprised ibuprofen, polyethylene oxide (PEO 301), glycine, sodium carbonate, flow agents and various tableting aids, in which PEO was present at a concentration of 25% by weight of ibuprofen, sodium carbonate was present at concentration of 25% by weight of the ibuprofen, and glycine was present at a concentration of 25% by weight of ibuprofen within a monolithic tablet.

| **Ex. 17** | **mg** |
|---|---|
| Ibuprofen | 400 |
| PEO 301 | 100 |
| Na2CO3 | 100 |
| Glycine | 100 |
| Stearic acid | 10 |
| **Total:** | **710** |

All ingredients were passed through a 30-mesh screen. The ibuprofen was blended with the formulation components in a V-blender. The resulting powder was compressed into tablets using conventional technologies.

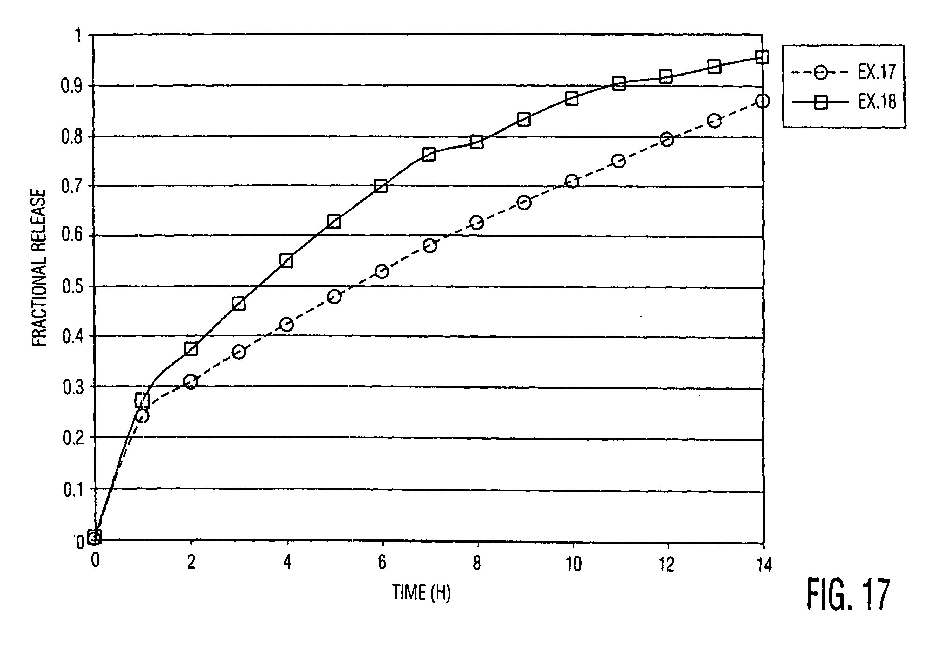

As shown in **Fig. 17**, the results of this Example demonstrate the invention is capable of an *in vitro* release profile comprising a burst effect, followed by the sustained release of the remaining material. The *in vitro* profile shows greater than 20 % release before 2.0 hours with a constant rate release and at least 80% release by 8 hours.

### Example 18 (not according to the invention)

In another embodiment, the formulation comprised ibuprofen, polyethylene oxide (PEO 301), glycine, sodium carbonate, croscarmellose sodium, flow agents and various tableting aids, in which PEO was present at a concentration of 25% by weight of ibuprofen, sodium carbonate was present at concentration of 25% by weight of the ibuprofen, and glycine was present at a concentration of 25% by weight of ibuprofen within a monolithic tablet.

| **Ex. 18** | **mg** |
|---|---|
| Ibuprofen | 400 |
| PEO 301 | 100 |
| Na2CO3 | 100 |
| Glycine | 100 |
| Croscarmellose Sodium | 50 |
| DCP | 150 |
| Stearic acid | 10 |
| **Total:** | **910** |

All ingredients were passed through a 30-mesh screen. The ibuprofen was blended with the formulation components in a V-blender. The resulting powder was compressed into tablets using conventional technologies.

As shown in **Fig. 17**, the results of this Example demonstrate the invention is capable of an *in vitro* release profile comprising a burst effect, followed by the sustained release of the remaining material. The *in vitro* profile shows greater than 20% release before 2.0 hours with a constant rate release and at least 90% release by 8 hours.

### Comparative in vitro data

BRUFEN RETARD is a commercially available in Europe as a sustained release formulation of ibuprofen. BRUFEN RETARD tablets are specially formulated to allow the gradual release of active substance giving stable levels and a prolonged duration of effect over the dosage interval. BRUFEN RETARD is a film coated tablet with 800mg of ibuprofen. BRUFEN RETARD is indicated for its analgesic and anti-inflammatory effect in the treatment of rheumatoid arthritis (including juvenile rheumatoid arthritis or Still's disease), ankylosing spondylitis, and osteo-arthritis. BRUFEN RETARD is indicated in the treatment of non-articular rheumatism including fibrositis. BRUFEN RETARD is indicated in periarticular conditions such as frozen shoulder (capsulitis), bursitis, tendinitis, tenosynovitis and low-back pain. BRUFEN RETARD can also be used in soft-tissue injuries such as sprains and strains. BRUFEN RETARD is also indicated for its analgesic effect in the relief of mild to moderate pain such as dysmenorrhoea, dental, post-episiotomy pain and post-partum pain.

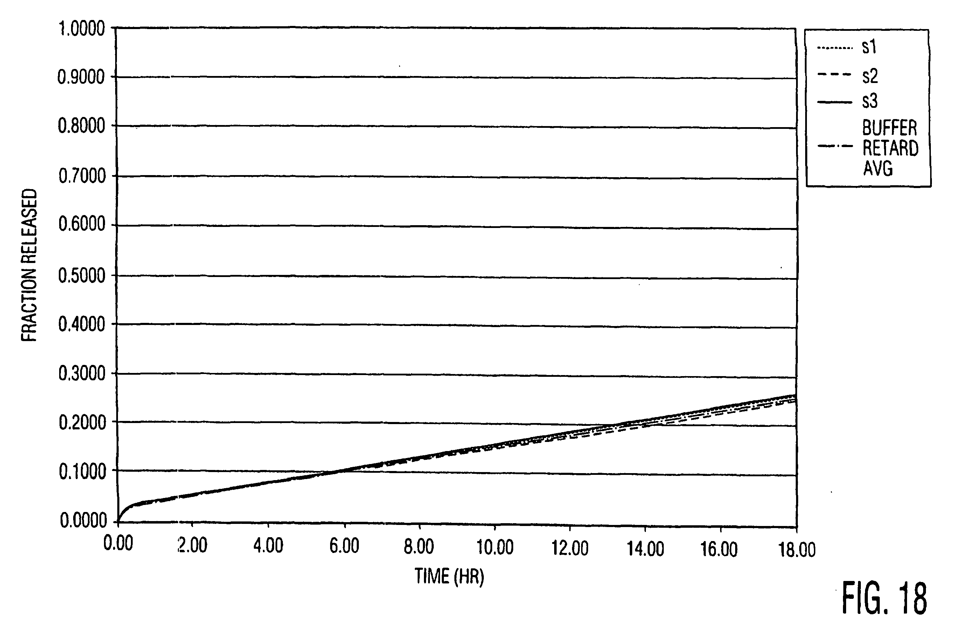

### Example 19 (Figure 18)

BRUFEN RETARD tablet *in vitro* release performance was evaluated in a type II dissolution apparatus in 900mL KH₂PO₄ buffer, pH 7.2, at 50rpm paddle speed.

As shown in **Fig. 18**, the results of this Example demonstrate the *in vitro* data results of BRUFEN RETARD. The figure shows that BRUFEN RETARD is incapable of an *in vitro* release profile comprising a burst effect, followed by the sustained release of the remaining material. BRUFEN RETARD fails to deliver to release at least 20% of ibuprofen by 2.0 hours with a constant rate of release with at least 70% release at 14 hours.

### Example 20 - In Vivo Trial

In the *in vivo* testing, serum concentrations of subjects taking tablets comprising the modified release formulation of the present invention were compared with serum concentrations of subjects taking immediate release ibuprofen tablets (Motrin^{®} IB 200 mg and Motrin^{®} 600 mg). Tablets comprising the modified release formulation of the present invention demonstrated a burst effect followed by sustained release and therapeutic concentration at extended time periods that the other two immediate release formulations did not. The minimum mean serum plasma ibuprofen concentration in the blood of the subject was between 8 and 10µg/ml for Motrin^{®} IB..

The *in vivo* behavior of modified release solid dosages of 1a and 1b from Example 1 were compared to the *in vivo* behavior of an immediate release formulation (MOTRIN^{®}). The open-label study involved 10 healthy male volunteers over the age of 18. Following an overnight fast of at least ten hours, each subject received either one 600 mg dose of one of the two above described modified release tablets or 200 mg every four hours for 3 doses of the immediate release formulation of MOTRIN^{®} IB or one 600 mg tablet of MOTRIN^{®}. 88 blood samples were taken prior to dosing and at specific intervals up to 12 hours after dosing.

The blood samples were kept in ice bath prior to centrifugation and were centrifuge as soon as possible under refrigerated condition at 35000 rpm for seven minutes. The collected plasma from each blood collection tube was aliquotted into precooled labeled polypropylene tubes. The samples were kept in an ice bath, then stored frozen at minus 25 °C ±10 °C until assayed.

The plasma samples were analyzed by a fully validated HPLC method. The analytes were separated by reverse phase chromatography. Evaluation of the assay was carried out by the construction of an eight point calibration curve (excluding zero concentration) covering the range of 0.400 µg/ml to 51.200 µg/ml (in human plasma) for ibuprofen. The slope and intercept of the calibration curves were determined through weighted linear regression analysis (1/conc.²). The results are depicted in FIGURE 19.

**Table 1. Summary of 90% CI**

| **Formulation** | **Reference: D (1 x 600 mg)** | | | **Reference: E (3 x 200 mg)** | | |
|---|---|---|---|---|---|---|
| | **Cₘₐₓ** | **AUC₀₋ₗₐₛₜ** | **AUCO_{-∞}** | **Cₘₐₓ** | **AUC₀₋ₗₐₛₜ** | **AUC₀₋ₗₐₛₜ** |
| B (1a) | 42.4-53.8 | 96.2-115 | 97.0-116 | 67.0-85.0 | 86.9-104 | 86.3-103 |
| C (1b) | 44.7-57.0 | 96.9-116 | 98.7-119 | 70.7-90.3 | 87.5-105 | 87.7-106 |
| D | - | - | - | 140-179 | 82.3-99.2 | 80.9-97.7 |
| E | 55.9-71.5 | 101-122 | 102-124 | - | - | - |

| | | | | | | |
|---|---|---|---|---|---|---|
| D is a 3 x 200mg MOTRIN^{®} IB E is a 1 x 600mg MOTRIN^{®} Treatments (B & C) versus Treatment E | | | | | | |

The systematic exposure to ibuprofen after the administration of the one 600 mg ibuprofen tablet 1a or 1b (Treatments B & C) was similar to that obtained when compared to the administration of one MOTRIN^{®} 600mg tablet. The peak exposure to ibuprofen from one 600 mg ibuprofen tablet 1a or 1b (Treatments A-C) was significantly lower than that from the MOTRIN^{®} 600mg tablet. The absorption time was modified comparing one 600 mg ibuprofen tablet 1a or 1b (Treatments B & C) with median Tₘₐₓ value of 5.0h to a 1.5h Tₘₐₓ of one MOTRIN^{®} 600mg tablet.

### Treatments (B & C) versus Treatment D

The systematic exposure to ibuprofen after the administration of the one 600 mg ibuprofen tablet 1a or 1b (Treatments B & C) was similar to that obtained when compared to the administration of three MOTRIN^{®} IB 200mg tablets. The peak exposure to ibuprofen from one 600 mg ibuprofen tablet 1a or 1b (Treatments B & C) was significantly lower than that from three MOTRIN^{®} IB 200mg tablets. The absorption time was modified comparing one 600 mg ibuprofen tablet 1a or 1b (Treatments B & C) with median Tₘₐₓ value of 5.0h to a 1.0h Tₘₐₓ of three MOTRIN^{®} IB 200mg tablet.

Figure 19 depicts the results discussed above. Treatment D shows an initial burst that falls to a valley at four hours and the second tablet is administered. This valley again happens at the eighth hour. This valley constitutes the minimum plasma concentration for ibuprofen to be considered therapeutic. A mean ibuprofen plasma concentration of about 6.4-10 µg/ml is considered the concentration of ibuprofen needed in the blood to be considered clinically effective. Treatment E shows an extreme initial burst of ibuprofen followed by a steady decline that falls below therapeutic threshold at about 6 hours.

Treatments B and C have an initial burst of ibuprofen that reaches the level of 6.4 µg/ml at about 0.5 to 1 hour and maintains the level until about hour 12. The present invention provides for a single dosage of ibuprofen that provides an initial burst similar to an immediate release formulation of ibuprofen and then provides a mean ibuprofen plasma concentration of above 6.4 µg/ml for about 12 hours.

## Claims

1. A solid dosage form for modified oral administration of ibuprofen comprising:
a hydrophilic polymer;
300 to 800 mg of ibuprofen in the solid dosage form uniformly dispersed in said polymer;
a dissolution additive dispersed in said hydrophilic polymer in an amount in the range of 10% to 35% by weight of the ibuprofen, said dissolution additive being sodium carbonate, glycine, arginine, croscarmellose sodium or a combination thereof, or a combination of any two of such dissolution additives; and
an inert formulation additive dispersed in said hydrophilic polymer in an amount in the range of 15% to 75% by weight of the ibuprofen, said formulation additive comprising microcrystalline cellulose, silica, magnesium stearate, stearic acid, lactose, pre-gelatinized starch, dicalcium phosphate or a combination of any of them,
wherein said hydrophilic polymer comprises a first hydroxypropyl methylcellulose having a viscosity of greater than 100 cps and a second HPMC having a viscosity of about 100 cps, each at a concentration of 17% to 42% by weight of ibuprofen,
wherein at least 20% of the ibuprofen is released within 2 hours following exposure to an agitated aqueous medium of a single dosage unit, then thereafter releases ibuprofen at a relatively constant rate over a period of at least 8 hours, and wherein at least 70% of the ibuprofen is released over a period of not more than 14 hours following such exposure,
wherein the release is determined using an agitated aqueous medium, stirring at 50 rpm in pH 7.2 KH₂PO₄ media.

2. The solid dosage form of claim 1, wherein ibuprofen is present in each dosage form in an amount of about 300 mg, 400 mg or 600 mg.

3. The solid dosage form of claim 1, where said inert formulation additive comprises microcrystalline cellulose present at a concentration at 17% to about 33% by weight of ibuprofen.

4. The solid dosage form of claim 3, wherein said inert formulation additive comprises a first microcrystalline cellulose having particle size of about 20 mm and a second MCC having particle size of about 180 mm, each of which is present at a concentration at 17% to about 33 % by weight of ibuprofen.

5. The dosage form of claim 1, being a tablet
wherein the hydrophilic polymer comprises hydroxypropyl methylcellulose at a concentration of 17% to 42% by weight of ibuprofen;
wherein ibuprofen is present in an amount of about 600 mg and dispersed uniformly in said polymer;
wherein said dissolution additive is sodium carbonate uniformly dispersed in said polymer; and
wherein said formulation additive is two differing particle sizes of microcrystalline cellulose dispersed in said polymer, each at 15% to 50% by weight of the ibuprofen.

6. The dosage form of claim 1, being a tablet
wherein the hydrophilic polymer comprises hydroxypropyl methylcellulose at a concentration of 17% to 42% by weight of ibuprofen;
wherein ibuprofen is present in an amount of about 600 mg and is dispersed uniformly in said polymer;
wherein said dissolution additive is glycine uniformly dispersed in said polymer at a concentration of 10% to 15% by weight of the ibuprofen.

7. The dosage form of claim 1, being a tablet
wherein the hydrophilic polymer comprises hydroxypropyl methylcellulose at a concentration of 17% to 42% by weight of ibuprofen;
wherein ibuprofen is present in an amount of about 600 mg and is dispersed uniformly in said polymer;
wherein said dissolution additive is glycine uniformly dispersed in said polymer at a concentration of 10% to 15% by weight of the ibuprofen;
wherein said formulation additive is two differing particle sizes of microcrystalline cellulose dispersed in said polymer, each at 15% to 50% by weight of the ibuprofen.

8. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein said dissolution additive is glycine uniformly dispersed in said polymer at a concentration of 5% to 35% by weight of the ibuprofen;
wherein the formulation additive is two differing particle sizes of microcrystalline cellulose dispersed in said polymer, each at 15% to 50% by weight of the ibuprofen.

9. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 300mg to 800mg ibuprofen dispersed uniformly in said polymer;
wherein the dissolution additive is glycine uniformly dispersed in said polymer at a concentration of 5% to 35% by weight of the ibuprofen and croscarmellose sodium uniformly dispersed in said polymer at a concentration of 1% to 15% by weight of the ibuprofen.

10. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 300mg to 800mg ibuprofen dispersed uniformly in said polymer;
wherein the dissolution additive is glycine uniformly dispersed in said polymer at a concentration of 5% to 35% by weight of the ibuprofen and croscarmellose sodium uniformly dispersed in said polymer at a concentration of 1% to 15% by weight of the ibuprofen;
wherein the formulation additive is two differing particle sizes of microcrystalline cellulose dispersed in said polymer, each at 15% to 50% by weight of the ibuprofen.

11. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 300mg to 800mg ibuprofen dispersed uniformly in said polymer;
wherein the dissolution additive is sodium carbonate uniformly dispersed in said polymer at a concentration of 5% to 35% by weight of the ibuprofen;
wherein the formulation additive is two differing particle sizes of microcrystalline cellulose dispersed in said polymer, each at 15% to 50% by weight of the ibuprofen.

12. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 300mg to 800mg ibuprofen dispersed uniformly in said polymer;
wherein the dissolution additive is sodium carbonate uniformly dispersed in said polymer at a concentration of 5% to 35% by weight of the ibuprofen, and croscarmellose sodium uniformly dispersed in said polymer at a concentration of 1% to 15% by weight of the ibuprofen;
wherein the formulation additive is two differing particle sizes of microcrystalline cellulose dispersed in said polymer, each at 15% to 50% by weight of the ibuprofen.

13. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 300mg to 800mg ibuprofen dispersed uniformly in said polymer;
wherein the dissolution additives are sodium carbonate uniformly dispersed in said polymer at a concentration of 5% to 35% by weight of the ibuprofen, glycine uniformly dispersed in said polymer at a concentration of 5% to 35% by weight of the ibuprofen and croscarmellose sodium uniformly dispersed in said polymer at a concentration of 1% to 15% by weight of the ibuprofen;
wherein the formulation additive is two differing particle sizes of microcrystalline cellulose dispersed in said polymer, each at 15% to 50% by weight of the ibuprofen.

14. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 300mg to 800mg ibuprofen dispersed uniformly in said polymer;
wherein the dissolution additive is sodium carbonate uniformly dispersed in said polymer at a concentration of 5% to 35% by weight of the ibuprofen, and croscarmellose sodium uniformly dispersed in said polymer at a concentration of 1% to 15% by weight of the ibuprofen;
wherein the formulation additive is two differing particle sizes of microcrystalline cellulose dispersed in said polymer, each at 15% to 50% by weight of the ibuprofen.

15. The dosage form of claim 1, being a tablet
wherein said hydrophilic polymer comprises hydroxypropyl methylcellulose having two differing viscosities, selected from the group consisting of HPMC 100 cps, HPMC 4000 cps, HPMC 15000 cps, and HPMC 100000 cps, each at a concentration of 17% to 42% by weight of ibuprofen;
wherein 600mg ibuprofen dispersed uniformly in said polymer;
wherein the dissolution additive is sodium carbonate uniformly dispersed in said polymer at a concentration of 10% to 35% by weight of the ibuprofen, and glycine uniformly dispersed in said polymer at a concentration of 1% to 15% by weight of the ibuprofen.

## Patentansprüche

1. Feste Dosierungsform zur modifizierten oralen Verabreichung von Ibuprofen, umfassend:
ein hydrophiles Polymer;
300 bis 800 mg Ibuprofen in der festen Dosierungsform, einheitlich dispergiert in dem Polymer;
ein Lösungsadditiv, dispergiert in dem hydrophilen Polymer, in einer Menge im Bereich von 10 Gew.-% bis 35 Gew.-% des Ibuprofens, wobei das Lösungsadditiv Natriumcarbonat, Glycin, Arginin, Croscarmellose-Natrium oder eine Kombination davon oder eine Kombination von jedweden zwei von solchen Lösungsadditiven ist; und
ein inertes Formulierungsadditiv, dispergiert in dem hydrophilen Polymer, in einer Menge im Bereich von 15 Gew.-% bis 75 Gew.-% des Ibuprofens, wobei das Formulierungsadditiv mikrokristalline Cellulose, Siliciumdioxid, Magnesiumstearat, Stearinsäure, Lactose, prägelierte Stärke, Dicalciumphosphat oder eine Kombination von jedweden davon umfasst,
wobei das hydrophile Polymer eine erste Hydroxypropylmethylcellulose mit einer Viskosität von höher als 100 cps und eine zweite HPMC mit einer Viskosität von etwa 100 cps jeweils in einer Konzentration von 17 Gew.-% bis 42 Gew.-% des Ibuprofens umfasst,
wobei mindestens 20 % des Ibuprofens innerhalb von 2 Stunden nach der Einwirkung eines bewegten wässrigen Mediums auf eine einzelne Dosierungseinheit freigesetzt wird, wobei dann danach Ibuprofen mit einer relativ konstanten Rate über einen Zeitraum von mindestens 8 Stunden freigesetzt wird und wobei mindestens 70 % des Ibuprofens über einen Zeitraum von nicht länger als 14 Stunden nach einer solchen Einwirkung freigesetzt wird, wobei die Freisetzung unter Verwendung eines bewegten wässrigen Mediums, wobei mit 50 UpM in KH₂PO₄-Medien, pH-Wert 7,2 gerührt wird, bestimmt wird.

2. Feste Dosierungsform nach Anspruch 1, wobei Ibuprofen in jeder Dosierungsform in einer Menge von etwa 300 mg, 400 mg oder 600 mg vorhanden ist.

3. Feste Dosierungsform nach Anspruch 1, wobei das inerte Formulierungsadditiv mikrokristalline Cellulose, welche in einer Konzentration von 17 Gew.-% bis etwa 33 Gew.-% des Ibuprofens vorhanden ist, umfasst.

4. Feste Dosierungsform nach Anspruch 3, wobei das inerte Formulierungsadditiv eine erste mikrokristalline Cellulose mit einer Teilchengröße von etwa 20 mm und eine zweite MCC mit einer Teilchengröße von etwa 180 mm umfasst, wobei beide in einer Konzentration von 17 Gew.-% bis etwa 33 Gew.-% des Ibuprofens vorhanden sind.

5. Dosierungsform nach Anspruch 1, welche eine Tablette ist,
wobei das hydrophile Polymer Hydroxypropylmethylcellulose in einer Konzentration von 17 Gew.-% bis 42 Gew.-% des Ibuprofens umfasst;
wobei Ibuprofen in einer Menge von etwa 600 mg und einheitlich dispergiert in dem Polymer vorhanden ist;
wobei das Lösungsadditiv Natriumcarbonat, einheitlich dispergiert in dem Polymer, ist;
und
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

6. Dosierungsform nach Anspruch 1, welche eine Tablette ist,
wobei das hydrophile Polymer Hydroxypropylmethylcellulose in einer Konzentration von 17 Gew.-% bis 42 Gew.-% des Ibuprofens umfasst;
wobei Ibuprofen in einer Menge von etwa 600 mg vorhanden ist und einheitlich in dem Polymer dispergiert ist;
wobei das Lösungsadditiv Glycin, einheitlich dispergiert in dem Polymer, in einer Konzentration von 10 Gew.-% bis 15 Gew.-% des Ibuprofens ist.

7. Dosierungsform nach Anspruch 1, welche eine Tablette ist,
wobei das hydrophile Polymer Hydroxypropylmethylcellulose in einer Konzentration von 17 Gew.-% bis 42 Gew.-% des Ibuprofens umfasst;
wobei Ibuprofen in einer Menge von etwa 600 mg vorhanden ist und einheitlich in dem Polymer dispergiert ist;
wobei das Lösungsadditiv Glycin, einheitlich dispergiert in dem Polymer, in einer Konzentration von 10 Gew.-% bis 15 Gew.-% des Ibuprofens ist;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

8. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei das Lösungsadditiv Glycin, einheitlich dispergiert in dem Polymer, in einer Konzentration von 5 Gew.-% bis 35 Gew.-% des Ibuprofens ist;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

9. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 300 mg bis 800 mg Ibuprofen einheitlich in dem Polymer dispergiert sind;
wobei das Lösungsadditiv Glycin, einheitlich dispergiert in dem Polymer, in einer Konzentration von 5 Gew.-% bis 35 Gew.-% des Ibuprofens und Croscarmellose-Natrium, einheitlich dispergiert in dem Polymer, in einer Konzentration von 1 Gew.-% bis 15 Gew.-% des Ibuprofens ist.

10. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 300 mg bis 800 mg Ibuprofen einheitlich in dem Polymer dispergiert sind;
wobei das Lösungsadditiv Glycin, einheitlich dispergiert in dem Polymer, in einer Konzentration von 5 Gew.-% bis 35 Gew.-% des Ibuprofens und Croscarmellose-Natrium, einheitlich dispergiert in dem Polymer, in einer Konzentration von 1 Gew.-% bis 15 Gew.-% des Ibuprofens ist;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

11. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 300 mg bis 800 mg Ibuprofen einheitlich in dem Polymer dispergiert sind;
wobei das Lösungsadditiv Natriumcarbonat, einheitlich dispergiert in dem Polymer, in einer Konzentration von 5 Gew.-% bis 35 Gew.-% des Ibuprofens ist;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

12. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 300 mg bis 800 mg Ibuprofen einheitlich in dem Polymer dispergiert sind;
wobei das Lösungsadditiv Natriumcarbonat, einheitlich dispergiert in dem Polymer, in einer Konzentration von 5 Gew.-% bis 35 Gew.-% des Ibuprofens und Croscarmellose-Natrium, einheitlich dispergiert in dem Polymer, in einer Konzentration von 1 Gew.-% bis 15 Gew.-% des Ibuprofens ist;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

13. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 300 mg bis 800 mg Ibuprofen einheitlich in dem Polymer dispergiert sind;
wobei die Lösungsadditive Natriumcarbonat, einheitlich dispergiert in dem Polymer, in einer Konzentration von 5 Gew.-% bis 35 Gew.-% des Ibuprofens, Glycin, einheitlich dispergiert in dem Polymer, in einer Konzentration von 5 Gew.-% bis 35 Gew.-% des Ibuprofens und Croscarmellose-Natrium, einheitlich dispergiert in dem Polymer, in einer Konzentration von 1 Gew.-% bis 15 Gew.-% des Ibuprofens sind;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

14. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 300 mg bis 800 mg Ibuprofen einheitlich in dem Polymer dispergiert sind;
wobei das Lösungsadditiv Natriumcarbonat, einheitlich dispergiert in dem Polymer, in einer Konzentration von 5 Gew.-% bis 35 Gew.-% des Ibuprofens und Croscarmellose-Natrium, einheitlich dispergiert in dem Polymer, in einer Konzentration von 1 Gew.-% bis 15 Gew.-% des Ibuprofens ist;
wobei das Formulierungsadditiv zwei unterschiedliche Teilchengrößen von mikrokristalliner Cellulose, dispergiert in dem Polymer, jeweils in 15 Gew.-% bis 50 Gew.-% des Ibuprofens, ist.

15. Dosierungsform nach Anspruch 1, welche eine Tablette ist,
wobei das hydrophile Polymer Hydroxypropylmethylcellulose mit zwei unterschiedlichen Viskositäten, ausgewählt aus der Gruppe, bestehend aus HPMC 100 cps, HPMC 4000 cps, HPMC 15000 cps und HPMC 100000 cps, jeweils in einer Konzentration von 17 Gew.-% bis 42 Gew.-% des Ibuprofens umfasst;
wobei 600 mg Ibuprofen einheitlich in dem Polymer dispergiert sind;
wobei das Lösungsadditiv Natriumcarbonat, einheitlich dispergiert in dem Polymer, in einer Konzentration von 10 Gew.-% bis 35 Gew.-% des Ibuprofens und Glycin, einheitlich dispergiert in dem Polymer, in einer Konzentration von 1 Gew.-% bis 15 Gew.-% des Ibuprofens ist.

## Revendications

1. Forme posologique solide pour l'administration orale modifiée d'ibuprofène comprenant :
un polymère hydrophile ;
300 à 800 mg d'ibuprofène sous la forme posologique solide dispersés uniformément dans ledit polymère ;
un additif de dissolution dispersé dans ledit polymère hydrophile dans une quantité dans la plage de 10% à 35% en poids de l'ibuprofène, ledit additif de dissolution étant du carbonate de sodium, de la glycine, de l'arginine, de la croscarmellose sodique, ou une combinaison de ceux-ci, ou une combinaison de deux quelconques de ces additifs de dissolution ; et
un additif de formulation inerte dispersé dans ledit polymère hydrophile dans une quantité dans la plage de 15% à 75% en poids de l'ibuprofène, ledit additif de formulation comprenant de la cellulose microcristalline, de la silice, de la silice, du stéarate de magnésium, de l'acide stéarique, du lactose, de l'amidon pré-gélatinisé, du phosphate dicalcique ou une combinaison de ceux-ci,
dans laquelle ledit polymère hydrophile comprend une première hydroxypropylméthylcellulose ayant une viscosité supérieure à 100 cps et une seconde HPMC ayant une viscosité d'environ 100 cps, chacune à une concentration de 17% à 42% en poids d'ibuprofène,
dans laquelle au moins 20% de l'ibuprofène sont libérés dans les 2 heures après l'exposition à un milieu aqueux agité d'une unité de dosage unique, puis ensuite l'ibuprofène est libéré à une vitesse relativement constante sur une période d'au moins 8 heures, et dans laquelle au moins 70% de l'ibuprofène sont libérés sur une période de pas plus de 14 heures après l'exposition,
dans laquelle la libération est déterminée en utilisant un milieu aqueux agité, en agitant à 50 tr/min dans un milieu à pH 7,2 de KH₂PO₄.

2. Forme posologique solide selon la revendication 1, dans laquelle l'ibuprofène est présent dans chaque forme posologique dans une quantité d'environ 300 mg, 400 mg ou 600 mg.

3. Forme posologique solide selon la revendication 1, dans laquelle ledit additif de formulation inerte comprend de la cellulose microcristalline présente à une concentration de 17% à environ 33% en poids d'ibuprofène.

4. Forme posologique solide selon la revendication 3, dans laquelle ledit additif de formulation inerte comprend une première cellulose microcristalline ayant une taille de particule d'environ 20 mm et une seconde MCC ayant une taille de particule d'environ 180 mm, dont chacune est présente à une concentration de 17% à environ 33% en poids d'ibuprofène.

5. Forme posologique selon la revendication 1, étant un comprimé
dans laquelle le polymère hydrophile comprend de l'hydroxypropylméthylcellulose à une concentration de 17% à 42% en poids d'ibuprofène ;
dans laquelle l'ibuprofène est présent dans une quantité d'environ 600 mg et est dispersé uniformément dans ledit polymère ;
dans laquelle ledit additif de dissolution est du carbonate de sodium dispersé uniformément dans ledit polymère ;
et
dans laquelle ledit additif de formulation est constitué de deux tailles de particules différentes de cellulose microcristalline dispersées dans ledit polymère, chacune à 15% à 50% en poids de l'ibuprofène.

6. Forme posologique selon la revendication 1, étant un comprimé
dans laquelle le polymère hydrophile comprend de l'hydroxypropylméthylcellulose à une concentration de 17% à 42% en poids d'ibuprofène ;
dans laquelle l'ibuprofène est présent dans une quantité d'environ 600 mg et est dispersé uniformément dans ledit polymère ;
dans laquelle ledit additif de dissolution est de la glycine dispersée uniformément dans ledit polymère à une concentration de 10% à 15% en poids de l'ibuprofène.

7. Forme posologique selon la revendication 1, étant un comprimé
dans laquelle le polymère hydrophile comprend de l'hydroxypropylméthylcellulose à une concentration de 17% à 42% en poids d'ibuprofène ;
dans laquelle l'ibuprofène est présent dans une quantité d'environ 600 mg et est dispersé uniformément dans ledit polymère ;
dans laquelle ledit additif de dissolution est de la glycine dispersée uniformément dans ledit polymère à une concentration de 10% à 15% en poids de l'ibuprofène ;
dans laquelle ledit additif de formulation est constitué de deux tailles de particules différentes de cellulose microcristalline dispersées dans ledit polymère, chacune à 15% à 50% en poids de l'ibuprofène.

8. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle ledit additif de dissolution est de la glycine dispersée uniformément dans ledit polymère à une concentration de 5% à 35% en poids de l'ibuprofène ;
dans laquelle l'additif de formulation est constitué de deux tailles différentes de cellulose microcristalline dispersées dans ledit polymère, chacune à 15% à 50% en poids de l'ibuprofène.

9. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 300 mg à 800 mg d'ibuprofène sont dispersés uniformément dans ledit polymère ;
dans laquelle l'additif de dissolution est de la glycine dispersée uniformément dans ledit polymère à une concentration de 5% à 35% en poids de l'ibuprofène et la croscarmellose sodique est dispersée uniformément dans ledit polymère à une concentration de 1% à 15% en poids de l'ibuprofène.

10. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 300 mg à 800 mg d'ibuprofène sont dispersés uniformément dans ledit polymère ;
dans laquelle l'additif de dissolution est de la glycine dispersée uniformément dans ledit polymère à une concentration de 5% à 35% en poids de l'ibuprofène et la croscarmellose sodique est dispersée uniformément dans ledit polymère à une concentration de 1% à 15% en poids de l'ibuprofène ;
dans laquelle l'additif de formulation est constitué de deux tailles différentes de cellulose microcristalline dispersées dans ledit polymère, chacune à 15% à 50% en poids de l'ibuprofène.

11. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 300 mg à 800 mg d'ibuprofène sont dispersés uniformément dans ledit polymère ;
dans laquelle ledit additif de dissolution est du carbonate de sodium dispersé uniformément dans ledit polymère à une concentration de 5% à 35% en poids de l'ibuprofène ;
dans laquelle l'additif de formulation est constitué de deux tailles différentes de cellulose microcristalline dispersée dans ledit polymère, chacune à 15% à 50% en poids de l'ibuprofène.

12. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 300 mg à 800 mg d'ibuprofène sont dispersés uniformément dans ledit polymère ;
dans laquelle l'additif de dissolution est du carbonate de sodium dispersé uniformément dans ledit polymère à une concentration de 5% à 35% en poids de l'ibuprofène et la croscarmellose sodique est dispersée uniformément dans ledit polymère à une concentration de 1% à 15% en poids de l'ibuprofène ;
dans laquelle l'additif de formulation est constitué de deux tailles différentes de cellulose microcristalline dispersée dans ledit polymère, chacune à 15% à 50% en poids de l'ibuprofène.

13. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 300 mg à 800 mg d'ibuprofène sont dispersés uniformément dans ledit polymère ;
dans laquelle les additifs de dissolution est du carbonate de sodium dispersé uniformément dans ledit polymère à une concentration de 5% à 35% en poids de l'ibuprofène, de la glycine dispersée uniformément dans ledit polymère à une concentration de 5% à 35% en poids de l'ibuprofène et la croscarmellose sodique est dispersée uniformément dans ledit polymère à une concentration de 1% à 15% en poids de l'ibuprofène ;
dans laquelle l'additif de formulation est constitué de deux tailles différentes de cellulose microcristalline dispersées dans ledit polymère, chacune à 15% à 50% en poids de l'ibuprofène.

14. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 300 mg à 800 mg d'ibuprofène sont dispersés uniformément dans ledit polymère ;
dans laquelle l'additif de dissolution est du carbonate de sodium dispersé uniformément dans ledit polymère à une concentration de 5% à 35% en poids de l'ibuprofène, et la croscarmellose sodique est dispersée uniformément dans ledit polymère à une concentration de 1% à 15% en poids de l'ibuprofène ;
dans laquelle l'additif de formulation est constitué de deux tailles différentes de cellulose microcristalline dispersées dans ledit polymère, chacune à 15% à 50% en poids de l'ibuprofène.

15. Forme posologique selon la revendication 1, étant un comprimé
dans laquelle ledit polymère hydrophile comprend de l'hydroxypropylméthylcellulose ayant deux viscosités différentes, sélectionnées dans le groupe constitué de HPMC 100 cps, HPMC 4000 cps, HPMC 15 000 cps, et HPMC 100 000 cps, chacune à une concentration de 17% à 42% en poids d'ibuprofène ;
dans laquelle 600 mg d'ibuprofène sont dispersés uniformément dans ledit polymère ;
dans laquelle l'additif de dissolution est du carbonate de sodium dispersé uniformément dans ledit polymère à une concentration de 10% à 35% en poids de l'ibuprofène, et de la glycine dispersée uniformément dans ledit polymère à une concentration de 1% à 15% en poids de l'ibuprofène.
